# FASCICULE DE BREVET EUROPEEN

(11) **EP 1 308 183 B1**
(45) Date de publication et mention de la délivrance du brevet: **25.02.2015**
(21) Numéro de dépôt: 02292682.8
(22) Date de dépôt: 29.10.2002
(51) Int. Cl.: A61N 1/368

(54) **Dispositif médical implantable actif pour le traitement des troubles du rythme cardiaque, comprenant des moyens perfectionnés de détection des arythmies auriculaires**
Aktive implantierbare medizinische Vorrichtung zur Behandlung von Herzrhythmusstörungen, mit verbesserten Mitteln zur Erkennung atrialer Arrhythmien
Active implantable medical device for treating heart rhythm disorders, having improved means for detecting atrial arrhythmias

(30) Priorité: 30.10.2001 FR 0113990
(43) Date de publication de la demande: 07.05.2003
(73) Titulaire: ELA MEDICAL, 92541 Montrouge (FR)
(72) Inventeur: Henry, Christine, 75014 Paris (FR); Limousin, Marcel, 75014 Paris (FR)
(74) Mandataire: Dupuis-Latour, Dominique

(56) Documents cités:
- EP-A- 1 072 284
- EP-A- 1 123 716
- WO-A-00/24460
- WO-A-00/47277
- US-A- 5 658 320

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs, ou encore les dispositifs "multisite", permettant de délivrer au coeur des impulsions électriques de faible énergie pour le traitement des troubles du rythme cardiaque.

L'invention sera principalement décrite dans le contexte d'un appareil implantable tel qu'un cardioverteur ou un défibrillateur, destiné à traiter les tachycardies et les tachyarythmies, mais elle peut être également appliquée à des appareils tels que les stimulateurs cardiaques, destinés à traiter les bradycardies, ces deux catégories pouvant d'ailleurs être combinées en un seul et même appareil.

L'invention concerne plus précisément le recueil des signaux résultant d'événements cardiaques auriculaires. En particulier, la sensibilité des circuits de détection des signaux issus de la cavité atriale est programmée à des valeurs élevées de sensibilité, c'est-à-dire à des valeurs basses de seuil de détection (typiquement de l'ordre de 0,4 mV). Cette sensibilité élevée est recherchée pour pouvoir recueillir et interpréter des signaux de faible amplitude de l'oreillette en cas de troubles du rythme auriculaire, terme générique qui recouvre diverses arythmies auriculaires telles que tachycardie, fibrillation, *flutter,* etc. troubles tous caractérisés, à la détection, par un rythme auriculaire anormal et rapide. La sensibilité atriale est un paramètre programmable, mais qui est généralement fixe, au moins au cours d'un même cycle cardiaque.

Cette sensibilité élevée, nécessaire, induit parfois des fausses détections (faux positifs), c'est-à-dire des détections dans la cavité atriale de signaux ne correspondant pas à des événements spontanés (dépolarisations) ou stimulés (application d'impulsions de stimulation sur l'oreillette). Typiquement, ces détections parasites résultent d'un phénomène connu sous le nom de "far-field" ou "FFS" (*Far-Field Sensing*), qui se produit lorsque le stimulateur détecte dans la cavité auriculaire un signal issu de la dépolarisation ventriculaire antérieure. En d'autres termes, la dépolarisation du ventricule se propage jusqu'à l'oreillette et, bien qu'atténuée, vient leurrer le circuit de détection auriculaire en raison de l'abaissement du seuil de sensibilité de ce dernier.

Un inconvénient d'une telle détection est qu'elle peut être interprétée par le dispositif comme une extrasystole auriculaire. Si ce phénomène est répétitif, l'appareil pourra l'interpréter comme une tachycardie atriale et déclencher en conséquence - à tort - des algorithmes de prévention ou de thérapie.

En d'autres termes, ces faux positifs peuvent conduire à un faux diagnostic d'association, lorsque le dispositif détecte - à tort - autant d'événements auriculaires (conséquences du far-field) que d'événements ventriculaires (à l'origine du far-field). Ce phénomène pénalise fortement la détection d'une dissociation du rythme (c'est-à-dire lorsque les événements auriculaires sont désynchronisés et en moins grand nombre que les événements ventriculaires) et, de façon générale, la détection des arythmies de toutes sortes.

Ainsi, l'un des buts de la présente invention est de proposer un dispositif implantable permettant d'éviter la détection, dans la chambre auriculaire, d'un signal engendré par une dépolarisation ventriculaire (far-field).

Un autre but de l'invention est, plus précisément, d'éviter de détecter un tel signal lors d'une tachycardie ventriculaire, souvent caractérisée par une succession d'événements ventriculaires non précédés d'un signal auriculaire véritable (rythme dissocié). En particulier, si le patient présente des événements ventriculaires non précédés d'une onde P (cas d'une extrasystole ventriculaire (ESV)), la détection de tels événements à l'étage auriculaire est préjudiciable au bon fonctionnement de l'implant.

À cet effet, l'invention propose un dispositif médical implantable actif comprenant des moyens de détection d'événements ventriculaires et auriculaires, des moyens de signalisation de la délivrance d'une impulsion de stimulation ventriculaire et/ou auriculaire, et des moyens pour inhiber les moyens de détection d'événements auriculaires, après détection d'un événement ventriculaire, pendant la durée d'une période réfractaire auriculaire absolue post-ventriculaire, les moyens de détection d'événements auriculaires comportant des moyens de protection à l'encontre de la détection de signaux auriculaires ne correspondant pas à un événement auriculaire effectivement survenu, notamment de signaux de far-field ventriculaire.

Les moyens de protection comportent des moyens d'ajustement dynamique de la sensibilité des moyens de détection des événements auriculaires, aptes à relever temporairement le seuil de détection, d'un premier incrément pendant une première durée, après détection par les moyens de détection ventriculaire d'un événement ventriculaire et détection par les moyens de détection auriculaire d'un événement auriculaire antérieur, spontané ou stimulé, au cours du même cycle.

Un tel dispositif est par exemple divulgué par le WO 00/24460 A1.

De façon caractéristique de l'invention, les moyens d'ajustement dynamique de la sensibilité sont également aptes à relever temporairement le seuil de détection, d'un second incrément pendant une seconde durée, après détection par les moyens de détection ventriculaire d'un événement ventriculaire sans détection par les moyens de détection auriculaire d'un événement auriculaire antérieur spontané au cours du même cycle.

Les premier et second incréments peuvent être de même valeur, par exemple comprise entre 0,2 et 0,6 mV, de préférence environ 0,4 mV. Les première et seconde durées peuvent être de même valeur, par exemple comprise entre 150 et 200 ms, de préférence environ 172 ms.

Selon un second aspect de l'invention, les moyens de protection comportent des moyens pour augmenter ladite période réfractaire auriculaire absolue post-ventriculaire d'une durée prédéterminée, ou jusqu'à une durée prédéterminée, après détection par les moyens de détection ventriculaire d'un événement ventriculaire et détection par les moyens de détection auriculaire d'un événement auriculaire antérieur stimulé au cours du même cycle.

La période réfractaire auriculaire absolue post-ventriculaire peut être par exemple augmentée d'une durée comprise entre 85 et 105 ms, de préférence environ 94 ms.

On va maintenant décrire un exemple de mise en oeuvre de l'invention, en référence aux dessins annexés.

La figure 1 est, pour un dispositif de l'art antérieur, une série de chronogrammes de divers signaux cardiaques montrant notamment les conséquences de la survenue d'extrasystoles ventriculaires et la détection de signaux de far-field.

La figure 2 est, pour un dispositif selon l'invention, une série de chronogrammes de divers signaux cardiaques montrant notamment la manière d'empêcher des faux diagnostics liés à la survenue d'extrasystoles ventriculaires et la détection de signaux de far-field.

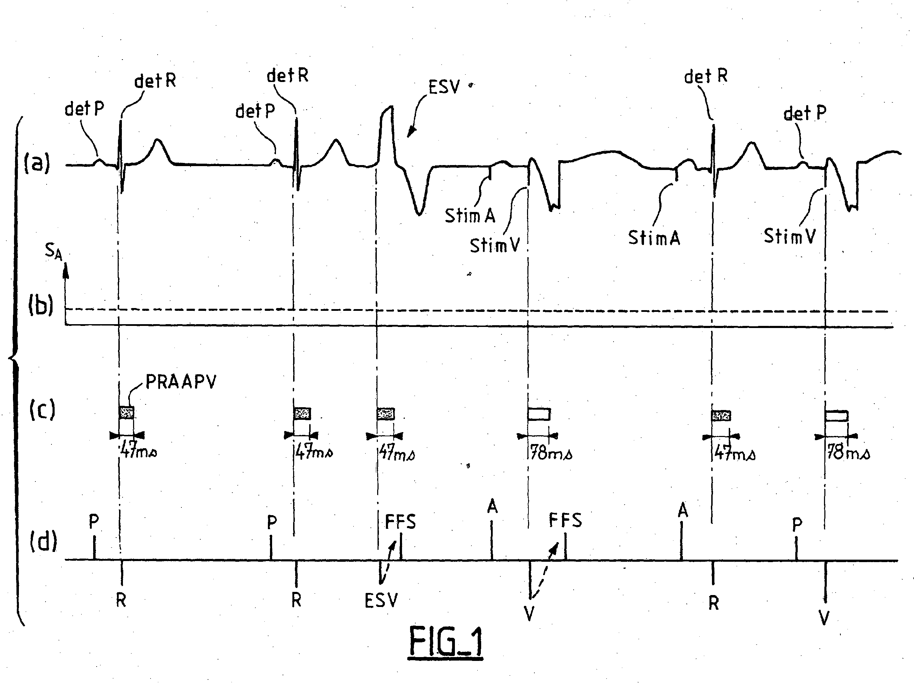

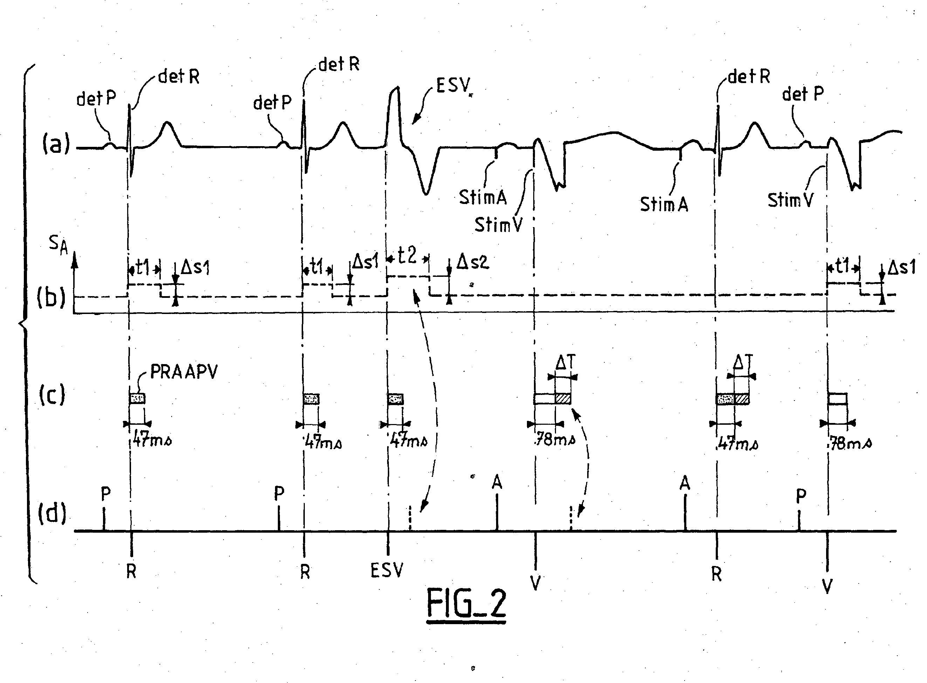

Sur les figures 1 et 2, le chronogramme (a) illustre un signal ECG sur lequel se succèdent divers événements cardiaques.

Par "événements", on entendra soit des signaux provenant de dépolarisations spontanées, soit des signaux résultant de stimuli appliqués par l'appareil. Ces événements sont référencés de la manière suivante :
- P ou detP : événement cardiaque auriculaire spontané (détecté) ;
- R ou detR : événement cardiaque ventriculaire spontané (détecté) ;
- A ou stimA : événement cardiaque auriculaire stimulé ;
- V ou stimV : événement cardiaque ventriculaire stimulé.

Les deux premiers cycles illustrés sur le chronogramme (a) correspondent à des cycles physiologiques, spontanés, suivis d'une extrasystole ventriculaire (ESV), puis de trois cycles stimulés avec : 1° stimulation auriculaire et ventriculaire, puis 2° stimulation auriculaire seule suivie d'une dépolarisation ventriculaire spontanée, enfin 3° dépolarisation auriculaire spontanée suivie d'une stimulation ventriculaire.

Le chronogramme (b) indique le niveau du seuil de détection auriculaire S_{A}, qui est fixe dans le cas de l'art antérieur (figure 1) et qui peut être temporairement augmenté dans le cas de l'invention (figure 2).

Sur la ligne (c), ont été repérées les périodes réfractaires auriculaires absolues post-ventriculaires (PRAAPV), périodes qui sont démarrées après tout événement ventriculaire (spontané ou stimulé) et qui viennent interdire toute détection d'un signal auriculaire pendant la durée de la période PRAAPV.

Dans l'exemple illustré, la PRAAPV a une durée de 47 ms dans le cas d'un événement ventriculaire spontané (R) et de 78 ms dans le cas d'un événement ventriculaire stimulé (V).

La ligne (d) présente la succession des marqueurs d'événements tels qu'analysés par le dispositif, tant auriculaires (P ou A, au dessus de la ligne) que ventriculaires (R ou V, au dessous de la ligne).

Si l'on considère maintenant l'analyse de l'ECG de la ligne (a) tel que vu par le dispositif, c'est-à-dire tel que représenté par les marqueurs de la ligne (d), on constate que la survenue de l'ESV, qui correspond à un signal d'amplitude importante, va induire la survenue d'un phénomène de far-field consécutif à l'ESV et qui sera recueilli par le circuit de détection auriculaire du dispositif et diagnostiqué comme un événement auriculaire (premier événement référencé FFS sur la ligne (d)) alors que ce signal n'est en fait qu'un "résidu" de l'ESV.

De la même façon, la stimulation ventriculaire intervenant au cycle qui suit immédiatement l'ESV va, elle aussi, induire une détection de far-field (deuxième événement référencé FFS sur la ligne (d)), interprétée à tort comme un événement auriculaire par le dispositif.

Dans ces conditions, pour tout signal ventriculaire un dispositif de l'art antérieur verra un signal auriculaire qui peut être interprété comme une onde P et fausser l'évaluation du degré d'association de l'événement ventriculaire suivant.

La figure 2 illustre la manière dont l'invention permet d'éviter ce faux diagnostic d'association, en repérant la succession d'événements ventriculaires non précédés d'un signal auriculaire associé, phénomène révélateur, notamment d'une tachycardie ventriculaire.

Pour cela, l'invention utilise deux techniques :
- La première technique consiste à relever provisoirement le seuil de détection atriale (SA), d'un incrément ΔS₁ pendant une durée t₁ en cas d'événement ventriculaire précédé d'une détection atriale dans le cycle, et à une valeur ΔS₂ pendant une durée t₂ en cas d'événement ventriculaire terminant un cycle sans détection ni stimulation atriale antérieure (ce qui correspond à la définition d'une ESV).
   Cette augmentation temporaire du seuil permet d'éviter la détection d'un signal de far-field consécutif à la survenue de l'ESV (signal représenté, pour mémoire, par le premier marqueur en pointillé sur le chronogramme de la ligne (d), juste après le marqueur correspondant à l'ESV).
   Les valeurs de seuil et d'augmentation de seuil sont avantageusement programmables, le seuil programmé étant par exemple de l'ordre de 0,4 mV, et les augmentations provisoires de seuil étant par exemple établies à ΔS₁ = ΔS₂ = 0,4 mV, pendant des durées t₁ = t₂ = 172 ms.
- L'autre technique consiste, en cas d'événement ventriculaire précédé d'une stimulation atriale dans le cycle, à augmenter la période réfractaire atriale PRAAPV d'une durée fixe ΔT (par exemple ΔT = 94 ms), ou à prolonger cette période réfractaire jusqu'à une durée prédéterminée.
   Cette augmentation de la période réfractaire masque la détection du far-field survenant lors du cycle suivant immédiatement l'ESV (signal représenté pour mémoire, par le second marqueur en pointillé sur la ligne (d)).

Le dispositif pourra ainsi détecter l'absence effective de signaux auriculaires associés à plusieurs événements ventriculaires, ce qui lui permettra ainsi de formuler rapidement un diagnostic de rythme dissocié si cette situation perdure ou se répète sur les cycles suivants.

## Revendications

1. Un dispositif médical implantable actif, tel qu'un stimulateur cardiaque, défibrillateur, cardioverteur et/ou dispositif multisite, comprenant :
- des moyens de détection d'événements ventriculaires (R, V) et auriculaires (P, A),
- des moyens de signalisation de la délivrance d'une impulsion de stimulation ventriculaire et/ou auriculaire, et
- des moyens pour inhiber les moyens de détection d'événements auriculaires, après détection d'un événement ventriculaire, pendant la durée d'une période réfractaire auriculaire absolue post-ventriculaire (PRAAPV),
les moyens de détection d'événements auriculaires comportant des moyens de protection à l'encontre de la détection de signaux auriculaires ne correspondant pas à un événement auriculaire effectivement survenu, notamment de signaux de far-field ventriculaire,
dispositif dans lequel les moyens de protection comportent des moyens d'ajustement dynamique de la sensibilité des moyens de détection des événements auriculaire, aptes à relever temporairement le seuil ) de détection, d'un premier incrément (ΔS₁) pendant une première durée (t₁), après détection par les moyens de détection ventriculaire d'un événement ventriculaire et détection par les moyens de détection auriculaire d'un événement auriculaire antérieur, spontané ou stimulé, au cours du même cycle,
**caractérisé en ce que** les moyens d'ajustement dynamique de la sensibilité des moyens de détection des événements auriculaires sont également aptes à relever temporairement le seuil de détection, d'un second incrément (ΔS₂) pendant une seconde durée (t₂), après détection par les moyens de détection ventriculaire d'un événement ventriculaire sans détection par les moyens de détection auriculaire d'un événement auriculaire antérieur spontané au cours du même cycle.

2. Le dispositif de la revendication 1, dans lequel lesdits premier et second incréments (ΔS₁, ΔS₂) sont de même valeur.

3. Le dispositif de la revendication 2, dans lequel la valeur desdits premier et second incréments (ΔS₁, ΔS₂) est comprise entre 0,2 et 0,6 mV, de préférence environ 0,4 mV.

4. Le dispositif de la revendication 1, dans lequel lesdites première et seconde durées (t₁, t₂) sont de même valeur.

5. Le dispositif de la revendication 4, dans lequel la valeur desdites première et seconde durées (t₁, t₂) est comprise entre 150 et 200 ms, de préférence environ 172 ms.

6. Le dispositif de la revendication 1, dans lequel les moyens de protection comportent des moyens pour augmenter ladite période réfractaire auriculaire absolue post-ventriculaire (PRAAPV) d'une durée prédéterminée (ΔT), ou jusqu'à une durée prédéterminée, après détection par les moyens de détection ventriculaire d'un événement ventriculaire et détection par les moyens de détection auriculaire d'un événement auriculaire antérieur stimulé au cours du même cycle.

7. Le dispositif de la revendication 6, dans lequel ladite période réfractaire auriculaire absolue post-ventriculaire (PRAAPV) est augmentée d'une durée (ΔT) comprise entre 85 et 105 ms, de préférence environ 94 ms.

## Patentansprüche

1. Aktive implantierbare medizinische Vorrichtung wie etwa ein Herzstimulator, Defibrillator, Kardioverter und/oder Mehrortvorrichtung, die Folgendes umfasst:
- Mittel zum Detektieren ventrikulärer Ereignisse (R, V) und aurikulärer Ereignisse (P, A),
- Mittel zum Signalisieren der Verabreichung eines ventrikulären und/oder aurikulären Stimulationsimpulses und
- Mittel zum Sperren der Mittel zum Detektieren aurikulärer Ereignisse nach der Detektion eines ventrikulären Ereignisses während der Dauer einer postventrikulären absoluten aurikulären Refraktionsperiode (PRAAPV),
wobei die Mittel zum Detektieren aurikulärer Ereignisse Mittel enthalten zum Schutz vor der Detektion von aurikulären Ereignissen, die keinem faktisch unerwartet auftretenden aurikulären Ereignis entsprechen, insbesondere von ventrikulären Fernfeldereignissen,
wobei in der Vorrichtung die Schutzmittel Mittel zum dynamischen Einstellen der Empfindlichkeit der Mittel zum Detektieren aurikulärer Ereignisse umfassen, die den Detektionsschwellenwert während einer ersten Dauer (t₁) nach der Detektion eines ventrikulären Ereignisses durch die Mittel für ventrikuläre Detektion und der Detektion eines späteren, spontanen oder stimulierten aurikulären Ereignisses während desselben Zyklus durch die Mittel für aurikuläre Detektion vorübergehend um ein erstes Inkrement (ΔS₁) anheben können,
**dadurch gekennzeichnet, dass** die Mittel zum dynamischen Einstellen der Empfindlichkeit der Mittel zum Detektieren aurikulärer Ereignisse außerdem den Detektionsschwellenwert während einer zweiten Dauer (t₂) nach der Detektion eines ventrikulären Ereignisses durch die Mittel für ventrikuläre Detektion ohne Detektion eines späteren, spontanen aurikulären Ereignisses während desselben Zyklus durch die Mittel für aurikuläre Detektion vorübergehend um ein zweites Inkrement (ΔS₂) anheben können.

2. Vorrichtung nach Anspruch 1, wobei die ersten und zweiten Inkremente (ΔS₁, ΔS₂) den gleichen Wert haben.

3. Vorrichtung nach Anspruch 2, wobei der Wert des ersten und des zweiten Inkrements (ΔS₁, ΔS₂) im Bereich von 0,2 bis 0,6 mV enthalten ist und vorzugsweise etwa 0,4 mV beträgt.

4. Vorrichtung nach Anspruch 1, wobei die erste und die zweite Dauer (t₁, t₂) den gleichen Wert haben.

5. Vorrichtung nach Anspruch 4, wobei der Wert der ersten und der zweiten Dauer (t₁, t₂) im Bereich von 150 bis 200 ms liegt und vorzugsweise etwa 172 ms beträgt.

6. Vorrichtung nach Anspruch 1, wobei die Schutzmittel Mittel umfassen, um die postventrikuläre absolute aurikuläre Refraktionsperiode (PRAAPV) um eine vorgegebene Dauer (ΔT) oder bis zu einer vorgegebenen Dauer zu erhöhen, nachdem ein ventrikuläres Ereignis durch die Mittel für ventrikuläre Detektion und ein späteres stimuliertes aurikuläres Ereignis während desselben Zyklus durch die Mittel für aurikuläre Detektion detektiert worden sind.

7. Vorrichtung nach Anspruch 6, wobei die postventrikuläre absolute aurikuläre Refraktionsperiode (PRAAPV) um eine Dauer (ΔT) erhöht wird, die im Bereich von 85 bis 105 ms liegt und vorzugsweise etwa 94 ms beträgt.

## Claims

1. An active implantable medical device, such as a pacemaker, defibrillator, cardioverter and/or multisite device, comprising:
- means for detecting ventricular (R, V) and atrial (P, A) events,
- means for signalling the delivery of a ventricular and/or atrial stimulation pulse, and
- means for inhibiting the atrial event detection means, after the detection of a ventricular event, during the duration of a post-ventricular absolute atrial refractory period (PRAAPV),
the atrial event detection means including means for protection against the detection of atrial signals that do not correspond to an atrial event having effectively occurred, in particular far-field ventricular signals,
device wherein the protection means include means for dynamically adjusting the sensitivity of the atrial event detection means, adapted to temporarily rise the detection threshold, by a first increment (ΔS₁) during a first duration (t₁), after detection by the ventricular detection means of a ventricular event and detection by the atrial detection means of a previous atrial event, spontaneous or stimulated, during the same cycle,
**characterized in that** the means for dynamically adjusting the sensitivity of the atrial event detection means are also adapted to temporarily rise the detection threshold, by a second increment (ΔS₂) during a second duration (t₂), after detection by the ventricular detection means of a ventricular event with no detection by the atrial detection means of a spontaneous previous atrial event during the same cycle.

2. The device of claim 1, wherein said first and second increments (ΔS₁, ΔS₂) are of same value.

3. The device of claim 2, wherein the value of said first and second increments (ΔS₁, ΔS₂) is comprised between 0.2 and 0.6 mV, preferably of about 0.4 mV.

4. The device of claim 1, wherein said first and second durations (t₁, t₂) are of same value.

5. The device of claim 4, wherein the value of said first and second durations (t₁, t₂) is comprised between 150 and 200 ms, preferably of about 172 ms.

6. The device of claim 1, wherein the protection means include means for increasing said post-ventricular absolute atrial refractory period (PRAAPV) by a predetermined duration (ΔT), or up to a predetermined duration, after detection by the ventricular detection means of a ventricular event and detection by the atrial detection means of a stimulated previous atrial event during the same cycle.

7. The device of claim 6, wherein said post-ventricular absolute atrial refractory period (PRAAPV) is increased by a duration (ΔT) comprised between 85 and 105 ms, preferably about 94 ms.
